# EUROPEAN PATENT SPECIFICATION

(11) **EP 3 610 873 B1**
(45) Date of publication and mention of the grant of the patent: **17.04.2024**
(21) Application number: 18785064.9
(22) Date of filing: 14.03.2018
(51) Int. Cl.: A61K 31/4375, A61K 31/4745, A61K 9/00, A61P 3/00

(54) **USE OF BERBERINE OR ACTIVE METABOLITE THEREOF IN THE PREPARATION OF A DRUG FOR PREVENTING AND/OR TREATING PHENYLKETONURIA**
VERWENDUNG VON BERBERIN ODER EINEM AKTIVEN METABOLITEN DAVON BEI DER HERSTELLUNG EINES ARZNEIMITTELS ZUR VORBEUGUNG UND BEHANDLUNG VON PHENYLKETONURIE
UTILISATION DE BERBÉRINE OU D'UN MÉTABOLITE ACTIF DE CELLE-CI DANS LA PRÉPARATION D'UN MÉDICAMENT POUR LA PRÉVENTION ET/OU LE TRAITEMENT DE LA PHÉNYLCÉTONURIE

(30) Priority: 11.04.2017 CN 201710231436
(43) Date of publication of application: 19.02.2020
(73) Proprietor: Institute of Materia Medica, Chinese Academy of Medical Sciences, Beijing 100050 (CN)
(72) Inventor: WANG, Yan, Beijing 100050 (CN); JIANG, Jiandong, Beijing 100050 (CN); ZHAO, Zhenxiong, Beijing 100050 (CN); MA, Shurong, Beijing 100050 (CN); SHOU, Jiawen, Beijing 100050 (CN); LI, Xiaoyang, Beijing 100050 (CN)
(74) Representative: Inspicos P/S
(86) International application number: PCT/CN2018/078910
(87) International publication number: WO 2018/188443

(56) References cited:
- WO-A1-98/00018
- WO-A1-03/090749
- WO-A1-2004/039372
- WO-A1-2006/029577
- CN-A- 103 372 210
- Naz Al Hafid ET AL: "Phenylketonuria: a review of current and future treatments", Translational pediatrics, 1 October 2015 (2015-10-01), pages 304-433607, XP055559701, China DOI: 10.3978/j.issn.2224-4336.2015.10.07 Retrieved from the Internet: URL:https://www.ncbi.nlm.nih.gov/pmc/artic les/PMC4728993/pdf/tp-04-04-304.pdf
- JIANG, ZHANGJIAN et al.: "Metabolic Profile Associated with Glucose and Cholesterol Lowering Effects of Berberine in Sprague-Dawley Rats", Metabolomics, vol. 8, no. 6, 22 February 2012 (2012-02-22), XP035131048,

## Description

### Technical field

The present invention refers to the application of berberine, or an active metabolite thereof, as claimed, or a pharmaceutically acceptable salt thereof in the preparation of a drug for preventing and/or treating phenylketonuria. It belongs to the technical field of medicinal technology.

### Background

Phenylketonuria (PKU) is a common amino acid metabolism disorder. It is caused by the defect of phenylalanine hydroxylase in the metabolic pathway of phenylalanine, making phenylalanine unable to be converted to tyrosine. This results in the accumulation of phenylalanine and phenylpyruvate and excreted in large amounts from the urine. Pediatric phenylketonuria is a common autosomal recessive disorder. The child is normal at birth, and the symptoms usually appear at 3 to 6 months after milk feeding starts. The symptoms become obvious at the age of 1 year. The main clinical features of phenylketonuria are mental retardation, mental and neurological symptoms, eczema, skin scratch marks, pigment loss, rat odor, and electroencephalographic abnormalities, etc. If early diagnosis and treatment are available, the aforementioned clinical manifestations may not occur. The intelligence will be normal, and the electroencephalographic abnormalities can be restored. At present, the most commonly used therapeutic method is to reduce the amount of phenylalanine in breast milk or infant formula. There is no drug for prevention/treatment in clinic.

Recent evidence suggests that the alterations in intestinal bacteria can affect the activities of central nervous system in animals, leading to the changes in the function and behavior of brain. The experimental results have supported the hypothesis of "gut-brain axis". However, the molecular mechanisms and chemical basis of the dialogue between host and bacteria remain unclear. It is very likely that intestinal bacteria regulate the function of brain through the production of metabolites by themselves which affect the biosynthesis of neurotransmitters, such as serotonin, and thereby affecting the physiological functions of host. Thus, clarifying the action mode of gut-brain pathway may help us understand the new functions of intestinal bacteria and facilitate the discovery of new methods to treat central nervous system disorders.

Berberine (BBR) is a natural compound which can be isolated from different kinds of plants, such as Coptis, Berberis, Goldenseal, and Cortex Phellodendri, etc. Berberine has been used as an over-the-counter drug to treat diarrhea without significant adverse effects in patients for several decades. Since 2004, we have discovered that berberine can be used as a new drug for the treatment of hyperlipidemia and type 2 diabetes. Its clinical efficacy has been confirmed by many research groups both at home and abroad. At the meantime, berberine has been shown to improve cognitive function and increase learning and memory in rodent models. The present patent describes that oral administration of berberine can reduce the level of blood phenylpyruvate while increase the level of tyrosine in o-methylphenylalanine induced phenylketonuria models of suckling rats, resulting in a down regulated ratio of phenylalanine/tyrosine. Accordingly, the levels of dopa and dopamine in brain after treatment are significantly increased in o-methylphenylalanine induced phenylketonuria models of SD suckling rats. While the intraperitoneal injection of berberine group and pseudo-germ-free animal oral berberine group have no obvious effect. Oral administration of berberine analogs, namely jatrorrhizine and dihydroberberine, can reduce the level of phenylpyruvate and the ratio of phenylalanine/tyrosine in blood of ICR mice as well.

Dopamine is one of the most important neurotransmitters in human and oral administration of berberine can increase the expression of dopa and dopamine in the brain of phenylketonuria models of suckling rats. At present, there is a lack of effective drugs for the treatment of phenylketonuria in children in clinic. Oral administration of berberine is able to improve the metabolic pathway of phenylalanine, increasing the biosynthesis of tyrosine from phenylalanine, and reducing the biotransformation of phenylpyruvate from phenylalanine. The increased tyrosine synthesis activates the synthesis pathway of dopa-dopamine, leading to a significant increase in the expression of dopamine in brain. Thus, the present patent describes that oral administration of berberine or an active metabolite thereof, or a pharmaceutically acceptable salt thereof, can reduce the level of phenylpyruvate and the ratio of phenylalanine/tyrosine in blood of SD suckling rats or ICR mice, thereby increasing the levels of dopa and dopamine in brain, suggesting their application in preventing and/or treating phenylketonuria. The mechanisms may be related to the gut-brain axis pathway under the regulation of intestinal bacteria.

### Summary of the Invention

The technical problem to be solved in the present invention is to provide a new drug for preventing and/or treating phenylketonuria.

To solve the technical problem in the present invention, the technical proposal is provided in the present invention as follows.

The present invention provides berberine represented by Formula (I), jatrorrhizine represented by Formula (II), or dihydroberberine represented by Formula (III), or pharmaceutically acceptable salts thereof for use in preventing and/or treating phenylketonuria, via oral administration,

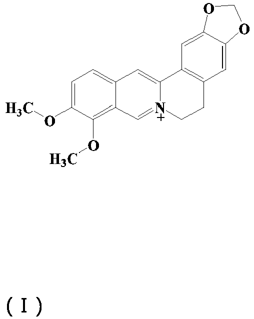

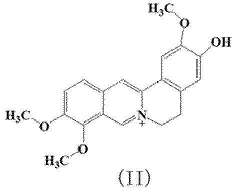

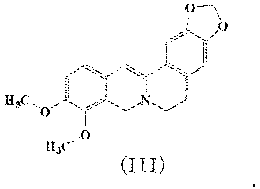

Said pharmaceutically acceptable salts include hydrochloride, sulfate, hydrobromate, hydroiodate, formate, acetate, or oxalate.

Said phenylketonuria mentioned is - in one aspect - caused by elevated phenylpyruvate in blood.

### Beneficial technical effect

Using o-methylphenylalanine induced phenylketonuria model of suckling rats is one of the important experiments in evaluating phenylketonuria.

α-Methylphenylalanine induced suckling rats are used, in which the concentration of phenylpyruvate in blood is significantly higher than that of the normal control group, and the ratio of phenylalanine/tyrosine is significantly higher than that in the normal control group. These results indicate that the metabolism of phenylalanine in suckling rats having phenylketonuria is abnormal and a large amount of phenylpyruvate is produced from phenylalanine, rather than tyrosine. The phenylpyruvate and the ratio of phenylalanine/tyrosine in blood of the suckling rats are significantly lower than that of the model group after oral administration of berberine. The oral administration of berberine can improve the metabolism of phenylalanine, and further prevent and/or treat the phenylketonuria.

### Description of Figures

Figure 1. Phenylpyruvate level in blood of suckling rats having phenylketonuria after treatment with berberine.
Figure 2. Phenylalanine/tyrosine ratio in blood of suckling rats having phenylketonuria after treatment with berberine.
Figure 3. Dopa level in brain of suckling rats having phenylketonuria after treatment with berberine.
Figure 4. Dopamine level in brain of suckling rats having phenylketonuria after treatment with berberine.
Figure 5. Tyrosine level in blood of ICR mice after oral administration of jatrorrhizine.
Figure 6. Phenylpyruvate level in blood of ICR mice after oral administration of jatrorrhizine.
Figure 7. Phenylpyruvate level in blood of ICR mice after oral administration of dihydroberberine.
Figure 8. Tyrosine level in blood of ICR mice after oral administration of dihydroberberine.

### Detailed Description of the Invention

Example 1. Therapeutic effect of berberine in o-methylphenylalanine induced phenylketonuria model of suckling rats.

The phenylpyruvate concentration and ratio of phenylalanine/tyrosine in blood are the key indicators for evaluating the therapeutic effect in phenylketonuria model of suckling rats.

### 1. Experimental animals, instruments and reagents

SD suckling rats (1 day old) were purchased from Beijing Vital River Laboratory Animal Technology Co., Ltd. All the animals and female rats were fed in SPF environment (21±2°C, 12-hour photoperiod) and had free access to food and water during the experiment. Berberine was purchased from the J&K Technology Co. LTD (Beijing, China). Phenylpyruvate, phenylalanine, and tyrosine were purchased from Solarbio Science&Technology Co. LTD (Beijing, China). α-Methylphenylalanine was purchased from Sinuokai Technology Co. LTD (Nanjing, China).

### 2. Experimental instruments and Analysis methods

High-performance liquid chromatography-triple quadrupole tandem mass spectrometry (LC-MS/MS 8050, Shimadzu Corporation, Japan) was used to quantitatively determine the concentration of dopa, phenylalanine, tyrosine, and phenylpyruvate. Alltima C18 (5 µm, 4.6 x 150 mm) chromatography column was used in the experiment, and the column temperature was maintained at 40 °C. The mobile phase to measure L-dopa was water-formic acid (100: 0.2 v/v) and acetonitrile. The condition of gradient elution was as follows (A: B, 0 min, 90:10; 2.5 min, 80:20; 2.51 min, 5:95; 5 min, 5:95; 5.01 min, 90:10; 8 min 90:10). The flow rate was 0.8 mL/min. Multi-reaction monitoring mode was used for quantization, and the quantitative ion pair of dopa was 198.15→152.05 (m/z). Phenylalanine, tyrosine, and phenylpyruvate level were determined using Alltima C18 (5 µm, 4.6 x 150 mm) chromatography column at column temperature of 40 °C. The mobile phase contained water-formic acid (100:0.1 v/v) and methanol. The condition of gradient elution was as follows (A: B, 0 min, 90:10; 1 min, 90:10; 1.01 min, 60:40; 5 min, 5:95; 7 min, 5:95; 7.01 min 90:10; 10 min 90:10). The flow rate was 0.8 mL/min. Multi-reaction monitoring mode was used for quantization. The quantitative ion pair was: phenylalanine, 165.85→120.20; tyrosine, 182.00→136.10; phenylpyruvate, 163.00→91.00.

### 3. Experimental design and animal grouping

Experimental design: The animals were divided into 6 groups, including: the normal control group, the model group, the low-dose of oral berberine treated model group, the high-dose of oral berberine treated model group, the intraperitoneal berberine treated model group, and the oral berberine treated pseudo-germ-free model group. The establishment of model started at 2 days old of SD suckling rats, which were administrated for 10 days, and then they were treated for 7 days.

Animal grouping:
(1) Normal control group: normal saline (s.c.).
(2) Model group: o-methylphenylalanine (s.c. 50 mg/kg/d), and phenylalanine (s.c. 200 mg/kg/d);
(3) Low-dose of oral berberine treated model group: o-methylphenylalanine (s.c. 50 mg/kg/d), phenylalanine (s.c. 200 mg/kg/d), berberine (oral, 100 mg/kg/d);
(4) High-dose of oral berberine treated model group: o-methylphenylalanine (s.c. 50 mg/kg/d), phenylalanine (s.c. 200 mg/kg/d), berberine (oral, 200 mg/kg/d);
(5) Intraperitoneal berberine treated model group: o-methylphenylalanine (s.c. 50 mg/kg/d), phenylalanine (s.c. 200 mg/kg/d), berberine (i.p. 20 mg/kg/d);
(6) Oral berberine treated pseudo-germ-free model group: o-methylphenylalanine (s.c. 50 mg/kg/d), phenylalanine (s.c. 200 mg/kg/d), berberine (oral, 200 mg/kg/d); Antibiotic dose: cefadroxil (100 mg/kg/d), oxytetracycline (300 mg/kg/d), erythromycin (300 mg/kg/d).

### 4. Results

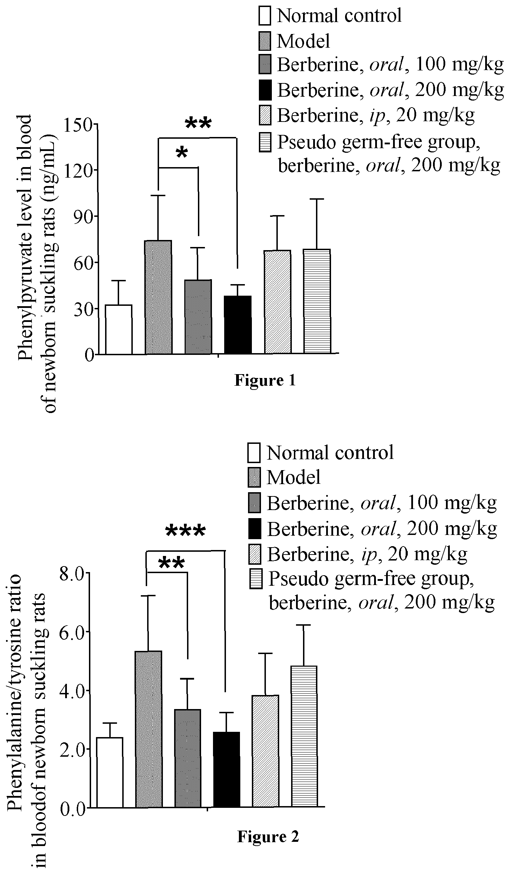

As shown in Figure 1, after 10 days of modeling, level of phenylpyruvate in blood of the model group of suckling rats was significantly higher than that of the normal control group, indicating that the model was established successfully. Phenylpyruvate level was significantly decreased in blood of suckling rats after oral treatment of berberine for 7 days, and the efficacy of berberine (oral, 100 and 200mg/kg/d) showed a dose-dependent manner. Phenylpyruvate level was not significantly changed in blood of the intraperitoneal injection treated group (20 mg/kg/d) and the pseudo-germ-free treated group (oral berberine at the same time), indicating that only oral treatment was effective, which may contribute to the stimulation of berberine to the intestinal bacteria.

As shown in Figure 2, the phenylalanine/tyrosine ratio in blood of the model group was significantly higher than that of the normal control group, indicating that the model was successfully established. Similarly, the phenylalanine/tyrosine ratio was significantly decreased in blood of suckling rats after oral treatment of berberine, showing a dose-dependent manner. The phenylalanine/tyrosine ratio was not significantly changed in blood of the intraperitoneal injection treated group and the pseudo-germ-free treated group.

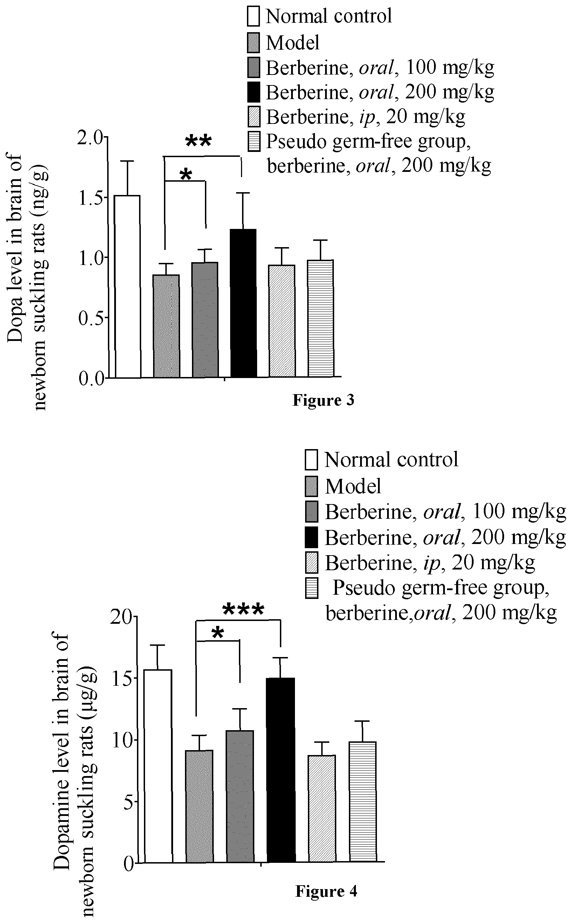

As shown in Figure 3, the concentration of dopa in brain of the model group was significantly lower than that of the normal control group, indicating that the metabolism of neurotransmitter was abnormal in brain of the model group of SD suckling rats. After oral treatment of berberine, dopa in brain of suckling rats significantly increased in a dose-dependent manner. The concentration of dopa was not significantly changed in brain of the intraperitoneal injection treated group and the pseudo-germ-free treated group.

As shown in Figure 4, the concentration of dopamine in brain of the model group was significantly lower than that of the normal control group, indicating that the metabolism of neurotransmitter was abnormal in brain of the model group of SD suckling rats. After oral treatment of berberine, dopamine in brain of suckling rats significantly increased in a dose-dependent manner. The concentration of dopamine was not significantly changed in brain of the intraperitoneal injection treated group and the pseudo-germ-free treated group.

### Example 2. Effect of Jatrorrhizine in reducing the level of phenylpyruvate in ICR mice.

The concentrations of phenylpyruvate and tyrosine in blood are the key indicators to evaluate the therapeutic effect for phenylketonuria in children.

### 1. Experimental animals, instruments and reagents

ICR mice (male, 20±2g) were purchased from Beijing Vital River Laboratory Animal Technology Co., Ltd. All the animals were fed in SPF environment (21±2°C, 12-hour photoperiod) and had free access to food and water during the experiment. Berberine was purchased from the J&K Technology Co. LTD (Beijing, China). Phenylpyruvate, phenylalanine, and tyrosine were purchased from Solarbio Science&Technology Co. LTD (Beijing, China).

### 2. Experimental design and animal grouping

Experimental design: The animals were divided into three groups, including the control group, the 200 mg/kg jatrorrhizine oral treated group at 12h, the 200 mg/kg jatrorrhizine oral treated group at 24h.

Animal grouping:
(1) The control group: saline 0.2mL (oral);
(2) The 200 mg/kg jatrorrhizine singe oral treated group at 12h: 200 mg/kg jatrorrhizine (oral);
(3) The 200 mg/kg jatrorrhizine single oral treated group at 24h: 200 mg/kg jatrorrhizine (oral).

### 3. Results

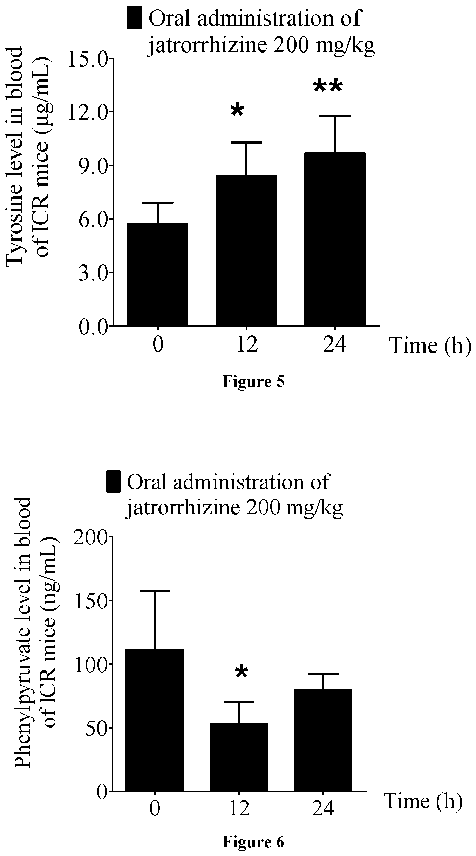

As shown in Figure 5, tyrosine level in blood of the ICR mice was significantly increased after oral administration of jatrorrhizine, indicating that jatrorrhizine could improve the metabolism of phenylalanine and it had the effect of treating phenylketonuria.

As shown in Figure 6, phenylpyruvate level in blood of the ICR mice was significantly decreased after oral administration of jatrorrhizine, indicating that jatrorrhizine could improve the metabolism of phenylalanine and it had the effect of treating phenylketonuria.

### Example 3. Effect of Dihydroberberine in reducing the level of phenylpyruvate in ICR mice.

The concentrations of phenylpyruvate and tyrosine in blood are the key indicators to evaluate the therapeutic effect for phenylketonuria.

### 1. Experimental animals, instruments and reagents

ICR mice (male, 20±2g) were purchased from Beijing Vital River Laboratory Animal Technology Co., Ltd. All the animals were fed in SPF environment (21±2°C, 12-hour photoperiod) and had free access to food and water during the experiment. Berberine was purchased from the J&K Technology Co. LTD (Beijing, China). Dihydroberberine was purchased from the Mansite Biology & Technology Co. LTD (Chengdu, China). Phenylpyruvate, phenylalanine, and tyrosine were purchased from Solarbio Science&Technology Co. LTD (Beijing, China).

### 2. Experimental design and animal grouping

Experimental design: The animals were divided into three groups, including the control group, the 200 mg/kg dihydroberberine oral treated group at 12h, the 200 mg/kg dihydroberberine oral treated group at 24h.

Animal grouping:
(1) The control group: saline 0.2mL (oral);
(2) The 100 mg/kg dihydroberberine singe oral treated group at 12h: 100 mg/kg jatrorrhizine (oral);
(3) The 100 mg/kg dihydroberberine singe oral treated group at 24h: 100 mg/kg jatrorrhizine (oral).

### 3. Results

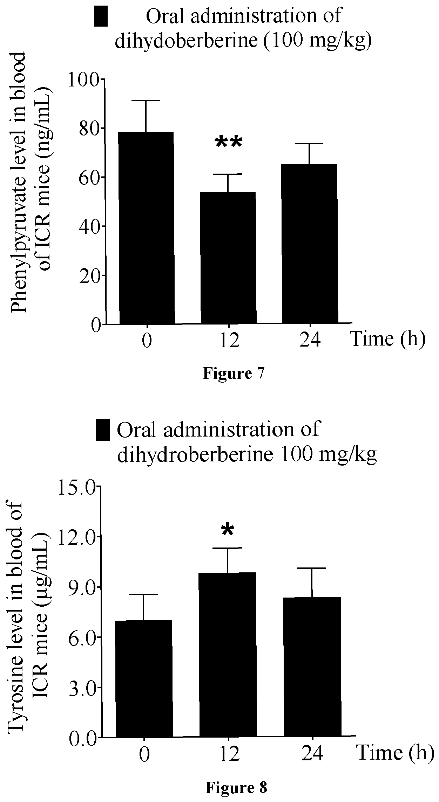

As shown in Figure 7, tyrosine level in blood of the ICR mice was significantly increased after oral administration of dihydroberberine, indicating that dihydroberberine could improve the metabolism of phenylalanine and it had the effect of treating phenylketonuria.

As shown in Figure 8, phenylpyruvate level in blood of the ICR mice was significantly decreased after oral administration of dihydroberberine, indicating that dihydroberberine could improve the metabolism of phenylalanine and it had the effect of treating phenylketonuria.

## Claims

1. Berberine represented by Formula (I), jatrorrhizine represented by Formula (II), or dihydroberberine represented by Formula (III), or pharmaceutically acceptable salts thereof for use in preventing and/or treating phenylketonuria, via oral administration,

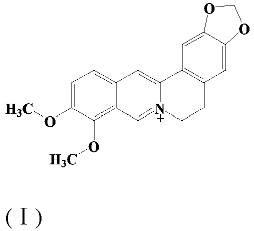

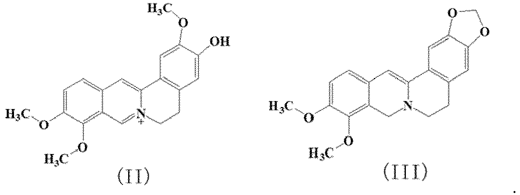

2. The substances according to claim 1 for use in preventing and/or treating phenylketonuria, wherein, said pharmaceutically acceptable salts include hydrochloride, sulfate, hydrobromate, hydroiodate, formate, acetate, or oxalate.

3. The substances according to claim 1 for use in preventing and/or treating phenylketonuria, wherein, said phenylketonuria is caused by elevated phenylpyruvate in blood.

## Patentansprüche

1. Berberin, dargestellt durch die Formel (I), Jatrorrhizin, dargestellt durch die Formel (II), oder Dihydroberberin, dargestellt durch die Formel (III), oder pharmazeutisch annehmbare Salze davon zur Verwendung bei der Vorbeugung und/oder Behandlung von Phenylketonurie durch orale Verabreichung.

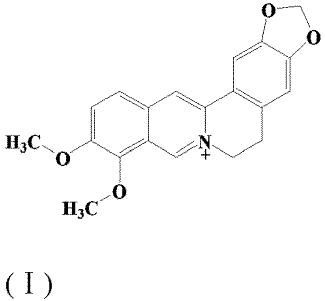

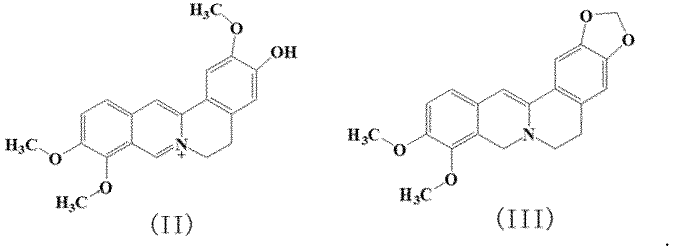

2. Substanzen nach Anspruch 1 zur Verwendung bei der Vorbeugung und/oder Behandlung von Phenylketonurie, wobei die pharmazeutisch annehmbaren Salze Hydrochlorid, Sulfat, Hydrobromat, Hydroiodat, Format, Acetat oder Oxalat umfassen.

3. Substanzen nach Anspruch 1 zur Verwendung bei der Vorbeugung und/oder Behandlung von Phenylketonurie, wobei die Phenylketonurie durch erhöhtes Phenylpyruvat im Blut verursacht wird.

## Revendications

1. Berbérine représentée par la formule (I), jatrorrhizine représentée par la formule (II) ou dihydroberbérine représentée par la formule (III) ou un de leurs sels pharmaceutiquement acceptables destinées à être utilisée dans la prévention et/ou le traitement de la phénylcétonurie, via une administration orale,

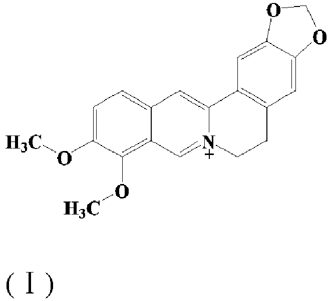

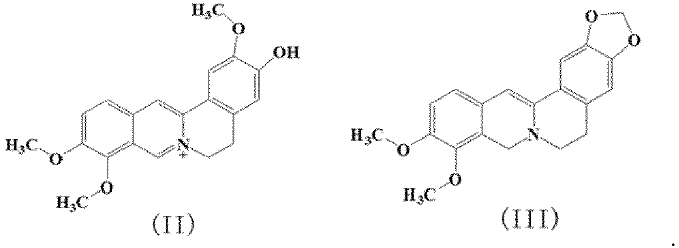

2. Substances selon la revendication 1, destinées à être utilisées dans la prévention et/ou le traitement de la phénylcétonurie, dans lesquelles, lesdits sels pharmaceutiquement acceptables comprennent le chlorhydrate, le sulfate, le bromhydrate, l'iodohydrate, le formate, l'acétate ou l'oxalate.

3. Substances selon la revendication 1 destinées à être utilisées dans la prévention et/ou le traitement de la phénylcétonurie, dans lesquelles, ladite phénylcétonurie est provoquée par du phénylpyruvate élevé dans le sang.
